Europäisches Patentamt

European Patent Office

Office européen des brevets

(11) Publication number: **0 242 243 B1**

(12) **EUROPEAN PATENT SPECIFICATION**

(45) Date of publication of patent specification: **23.06.93**

(51) Int. Cl.5: **C12N 15/62**, C12N 1/20, C07K 15/04, G01N 33/53, //(C12N1/20,C12R1:19)

(21) Application number: **87400504.4**

(22) Date of filing: **06.03.87**

(54) **Procedure for exposing an epitope within a protein possessing a distinct polypeptide structure, and the products obtained.**

(30) Priority: **07.03.86 FR 8603322**
**12.02.87 US 13796**

(43) Date of publication of application:
**21.10.87 Bulletin 87/43**

(45) Publication of the grant of the patent:
**23.06.93 Bulletin 93/25**

(84) Designated Contracting States:
**AT BE CH DE ES FR GB GR IT LI LU NL SE**

(56) References cited:
**EP-A- 0 146 416**

**JOURNAL OF IMMUNOLOGICAL METHODS, vol. 84, nos. 1-2, 28th November 1985, pages 53-63, Elsevier Science Publishers B.V. (Biomedical Division); J.-L. GUESDON et al.: "In situ enzyme immunodetection of surface or intracellular bacterial antigens using nitrocellulose sheets"**

(73) Proprietor: **INSTITUT PASTEUR**
**25-28, rue du Docteur Roux**
**F-75724 Paris Cédex 15(FR)**

Proprietor: **CENTRE NATIONAL DE LA RECHERCHE SCIENTIFIOUE (CNRS)**
**15, Ouai Anatole France**
**F-75700 Paris Cedex 07(FR)**

(72) Inventor: **Hofnung, Maurice**
**19 bis, rue Bargue**
**F-75015 Paris(FR)**
Inventor: **Charbit, Alain**
**97 avenue de Verdun**
**F-92130 Issy-les-Moulineaux(FR)**
Inventor: **Boulain, Jean-Claude**
**258, rue de Paris**
**F-91120 Palaiseau(FR)**

(74) Representative: **Gutmann, Ernest**
**S.C. Ernest Gutmann - Yves Plasseraud 67, boulevard Haussmann**
**F-75008 Paris (FR)**

Rank Xerox (UK) Business Services
(3.10/3.6/3.3.1)

EP 0 242 243 B1

PROCEEDINGS OF THE NATIONAL ACADEMY
OF SCIENCES USA, vol. 80, August 1983,
pages 5080-5084; S. VAN DER WERF et al.:
"Localization of a poliovirus type 1 neutral-
ization epitope in viral capsid polypeptide
VP1"

CHEMICAL ABSTRACTS, vol. 106, no. 9, 2nd
March 1987, page 245, abstract no. 29661j,
Columbus, Ohio, US; A. CHARBIT et al.:
"Probing the topology of a bacterial mem-
brane protein by genetic insertion of a for-
eign epitope; expression at the cell sur-
face", & EMBO J., 1986, 5(11), 3029-37

## Description

The invention relates to a genetic method for inserting an epitope into a protein to which it is normally foreign under conditions which allow the properties characteristic of the epitope, its immunogenic properties for example, to be expressed. One of the uses for which the invention is intended and which will be mentioned as an illustration is the incorporation of such an epitope in a carrier protein under conditions which confer on the hybrid protein formed immunogenic properties similar to those to which the epitope gives expression in the naturally occurring antigen of which it normally forms part. The aim to which the methods concerned are directed is well known. They relate to the preparation of selective vaccines, for example, the immunogenicity of which can be controlled and which are free of harmful side effects, unrelated to the effect desired of the vaccine.

Descriptions exist in the literature of numerous peptide sequences which are regarded as carriers of epitopes responsible for the known or recognized immunogenic effect of certain proteins so that attempts have already been made to isolate these epitopes, and, indeed, to synthesize a peptide containing them. One of the difficulties which was encountered when such peptides were used to constitute a vaccine lies, for example, in their lack of intrinsic immunogenicity, as a result, in particular, of their low molecular weight and a conformation which is not always the same as that adopted by the epitope when it forms part of the naturally occuring protein. It has therefore been suggested that such synthetic peptides (or fragments of naturally occurring proteins) be attached to high molecular weight supports.

Another solution which has already been suggested consists in the transformation of non-pathogenic bacteria, certain strains of E. coli for example, with appropriate vectors containing a nucleotide sequence coding for a protein normally exposed on the surface of these bacteria, the sequence having nonetheless been modified by the insertion of a fragment coding for the peptide which contains the desired epitope. This technique, the aim of which is to produce "live vaccines", combines several advantages : the possibility of producing them in large quantity, the reconstitution of a support exposing the epitope under conditions which facilitate its recognition by the immune system of the organisms for which the "live vaccine" is designed and, finally, the absence of pathogenicity of the said "live vaccine".

Bacteria of this type and the vectors appropriate to their production have been described in the French patent N° 82 16316,, filed 28th September 1982 and in the French patent N° 83 14236, filed 6th September 1983. These patents describe strains of micro-organisms, more especially strains of E. coli, transformed by vectors modified by at least part of the lamB gene, the latter being modified in turn by the insertion of a sequence constituted by a nucleotide fragment coding for a defined sequence of amino acids. The latter could, of course, be constituted by a sequence coding for a specific epitope. The aim of the inventions described in these patents was, in fact, to expose a hybrid protein containing the epitope concerned at the surface of the transformed E. coli cells and in such a position as to allow the immunogenicity of the epitope to be expressed.

The difficulties encountered have already been mentioned in the second of the patents cited above, even though the improved technique which it describes made it possible to reduce them appreciably. The transcription and translation of the entire, inserted nucleotide sequence were difficult to achieve. The hybrid protein synthesized were highly toxic for the host bacteria, a toxicity which was liable to entail lysis of a considerable proportion of the cells in culture.

The difficulty of producing mixed vaccines by recombining several distinct epitopes within one and the same protein has also been encountered in other areas. The problems associated with attaining appropriate exposure of an epitope inserted in a carrier protein to which it is normally foreign arise in the same way. In addition, the introduction of the epitope often entails a change in the overall conformation of the carrier protein, such that the intrinsic immunogenic properties of the latter are found to be affected by it. Sometimes the difficulty has been overcome by determining the most appropriate site for the insertion of the epitope into the peptide sequence of the receptor protein. However, the determination of a site in this way cannot be transposed from one protein to another, whereby the difficulties must be faced again every time the problem arises with regard to a new carrier protein. Another difficulty is often added to the preceding ones, at the level of the production of vectors which are subsequently used for producing hybrid proteins (at least when recourse is had to the techniques of genetic engineering to produce them). This additional difficulty lies in introducing the sequence coding for the epitope in the same reading frame as that of the corresponding parts of the sequence coding for the receptor protein.

The aim of the present invention is to suggest a different, general approach to this problem by the appropriate exposure of an epitope within a carrier protein, or, more generally, within a structure to which it is normally foreign such that the immunogenic properties of this epitope are expressed while at the same time the properties of the surrounding structure are kept under control. For instance such keeping under control means the preservation of at least some of the essential initial properties of the carrier protein.

This approach will be better understood if the particular problems posed by the exposure of a chosen epitope on the surface of E. coli bacteria are re-examined, on the understanding that this example must not be considered as being restrictive.

First, it should be remembered that the lamB protein, which will be used in the present example as the carrier of the peptide antigen to the surface of strains of E. coli bacteria, is normally located in the external membrane of E. coli. It is a transmembrane protein which is implicated in the transport of maltose and maltodextrins (for a review, see : HENGGE and BOOS, 1983), and acts as receptor for the adsorption of different bacteriophages, including the bacteriophage λ (CHARBIT and HOFNUNG, 1985). The sequence of lamB, the structural gene for this protein, has been determined (CLEMENT and HOFNUNG, 1981). It codes for a precursor of 446 amino acids which contain a signal peptide of 25 amino acids at its N-terminus. This peptide is cleaved when the protein is exported to the surface of the bacterium.

In practice, a DNA fragment which specifies the desired epitope has to be inserted into the lamB gene. In order that the aim of the invention be achieved this fusion must meet at least three conditions. (in such instance the suitable exposure of the epitope without modification of the essential properties of the lamB protein).

1° The hybrid protein thus constructed must be stable and not be toxic for the bacterium.

2° The fusion must be such that it does not destroy the instructions contained in the structural gene relating to the normal exportation of the protein.

3° The folding of the hybrid protein in the external membrane must be such that the epitope chosen be exposed on the surface of the bacterium and be clearly displayed.

First, a plasmid was constructed in which the lamB gene was placed under the control of a powerful but controllable promoter (tac) (AMANN et al., 1983). This plasmid also carries the structural gene of the repressor (lacI) which is able to control this promoter (BAGDASARIAN et al., 1983). Vectors of the same type have been described in the French patent n° 83 14236 cited above. The implementation of the procedure according to the invention, defined later in a general manner, has made it possible to define sites on the protein at which it is possible to insert a peptide sequence containing the epitope without disturbing the exportation or the folding of the protein. For that purpose, the plasmid was opened at random by a method already described which enables different plasmid molecules to be opened at sites distributed at random along their nucleotide chains (HEFFRON et al., 1978). Subsequently, it was closed in the presence of an excess of an adaptor (LATHE et al., 1984) of 10 base pairs which specify a site for the restriction enzyme BamHI. The importance of using an adaptor lies in its being subsequently possible to easily cleave the DNA at the site at which the adaptor was inserted and add an additional insert.

In the context of the example which will be described later, about 400 clones were analysed in which the adaptor had been inserted at random. 4 clones were selected in which the insertion of the adaptor had been performed with the preservation of the reading frame of translation of the lamB gene. In the context of this example, it was determined upon sequencing of the sequences coding for lamB, after their modification by the said adaptors, that in the clones selected insertion had occurred at the 146th, 153th, 189th and 374 th amino acids, respectively, from the N-terminus of the mature lamB protein. In three out of the four cases (sites 146, 153, 374) the presence of the adaptor was seen to correspond to the insertion of 4 amino acids. In the fourth case (site 189) 8 amino acids had been deleted and 5 amino acids had been inserted. In all cases, the hybrid proteins were stable and could be detected after electrophoresis on SDS-polyacrylamide gel, transfer to nitrocellulose filters and immunological detection by anti-lamB antiserum. Three of the hybrid proteins (sites 146, 153 and 374) still confer on the bacterium sensitivity to at least one phage which makes use of the λ receptor and the capacity to grow on dextrins. This strongly suggests that the folding of the hybrid protein in the external membrane is close to that of the wild type protein. Furthermore, these three hybrid proteins were not toxic for the bacterium : exponential growth was not affected by the presence of $10^{-3}$ molar isopropyl-ß-thiogalactoside (IPTG). In the fourth case (insertion at site 189) the hybrid protein was somewhat toxic for the bacterium (demonstrated by the inhibition of growth by the presence of IPTG), and no longer conferred sensitivity to phage on the transformed bacteria.

Of the four insertions which were prepared three have no appreciable effect on either the expotation or the folding of the lamB protein (sites 146, 153, 374). In addition, it is quite remarkable to note that, in the engineered products, the insertion sites correspond to regions of the protein which, according to the model proposed by CHARBIT et al., 1984, are localized at the outside of the membrane and on the bacterial surface. The fourth site (position 189) is situated inside of the outer membrane according to the same model.

The first three sites were chosen for the insertion of an additional peptide with a view to preparing new protein hybrids which expose the inserted peptide at the bacterial surface. These products of genetic engineering and their properties are described later.

A peptide of 11 amino acids which corresponds to the epitope 93-103 of the VP1 protein of poliovirus type 1 (HOGLE et al., 1985) was chosen as the additional peptide. This choice was dictated by the following considerations. The peptide is small. It is hydrophilic and was not be expected to function as stop sequence for the exportation of the hybrid protein. Polyclonal antibodies to the synthetic peptides corresponding to this region (anti-VP1) and a monoclonal antobody directed against the same region in the virus (anti C3) are available. In addition, the anti-C3 antibody has neutralizing properties (VAN DER WERF et al., 1983 ; WYCHOWSKI et al. 1983). The products of genetic engineering thus designed are thus likely to have medical importance.

An oligonucleotide specifying this peptide and terminated by BamHI cohesive ends was synthesized. The synthesis was performed so that the insertion of this oligonucleotide at the BamHI sequences at the sites 146, 153 and 374 preserves the reading frame for translation of the hybrid proteins. The three insertions were caried out by using the same oligonucleotide since the three adaptors inserted initially were all in the same phase.

After insertion of the oligonucleotide, three new hybrid proteins 146 VPI, 153VPI and 374VPI redesignated hereafter as 146C3, 153C3 and 374C3 were obtained. These three proteins were stable and also not toxic for the bacterium : exponential growth of the cells was not affected by the presence of $10^{-3}$ molar IPTG. After electrophoresis and transfer to nitrocellulose filters they reacted with a polyclonal anti-lamB antibody under denaturating conditions and by immunoprecipitation in non-denaturating conditions. This last property provided a demonstration of the fact that the hybrids were capable of adopting a trimeric structure just like the wild type lamB protein.

It is also interesting to note that the hybrid proteins 153C3 and 374C3 were capable of conferring on the bacteria sensitivity to at least one phage which makes use of the λ receptor, and the capacity to use dextrins as carbon source. Thus, in spite of the insertion, they had preserved most of the functions of the wild type lamB protein.

The capacity of these hybrid proteins to react with anti-VP1 antiserum (polyclonal) and anti-C3 antiserum (monoclonal) was then studied. Three experimental conditions were used : electrophoresis on SDS-polyacrylamide gel of denatured bacterial extracts, followed by transfer to nitro-cellulose filters and immunological detection (named hereafter immuno-blot), immunoprecipitation followed by electrophoresis in an SDS-polyacrylamide gel under non-denaturating conditions (named hereafter immuno-precipitation), immunological detection in situ on colonies after transfer of the colonies to nitrocellulose filters (named hereafter in situ dtection) (GUESDON et al., 1985). The main results were as follows.

All three of the hybrid proteins 146C3, 153C3 and 374C3 reacted with anti-VP1 antiserum to give an immuno-blot (under denaturating conditions).

The proteins 153C3 and 374C3 both reacted with the anti-VP1 and anti-C3 antobodies (by immunoprecipitation under non-denaturating conditions). Under the same conditions the hybrid protein 146C3 reacted weakly with the anti-VP1 antibody and showed no detectable reaction with anti-C3 antibody.

The protein hybrid 153C3 reacted in in situ detection with anti-C3 antibody without lysis (and hence all the more so after lysis with chloroform). The same was true for the hybrid protein 374C3, but in this case detection was better after lysis with chloroform. The epitope was thus recognized in both cases by the anti-C3 antibody and accessible to the latter on the surface of the cells. This result was confirmed for 153C3 and 374C3 by immunofluorescence under the optical microscope and under the electron microscope by means of protein A coupled to gold beads. The anti-VP1 antibody reacted in in situ detection with bacteria possessing the protein 153C3 but not with those carrying the protein 374C3. Bacteria producing the other hybrid protein 146C3 were not recognized by either of the two immune antisera in in situ detection (before and after lysis).

The characteristics of the different hybrids (hybrid proteins of lamB and C3, on the one hand, and the corresponding coding sequences, on the other) are presented in the Table which follows.

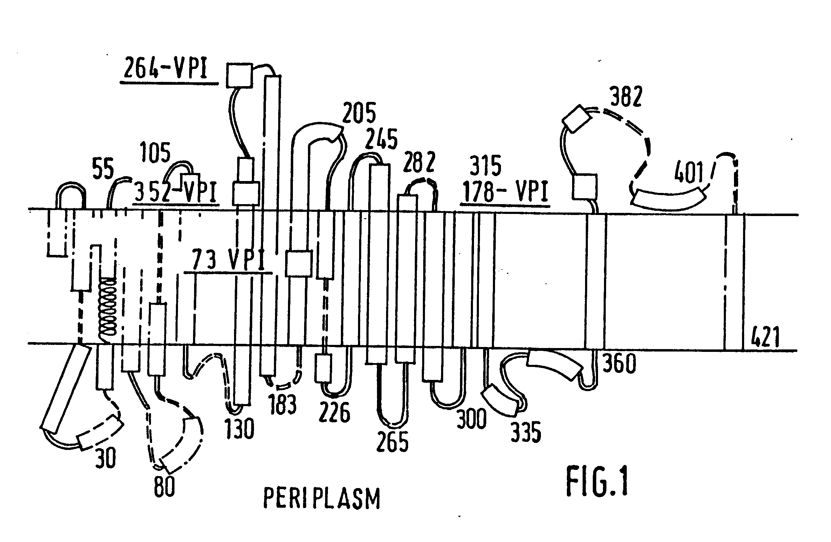

The model proposed by CHARBIT et al. referred to above is illustrated in Figure 1 which is appended. It provides a diagrammatic representation of the bactrial cell wall and, folded across this membrane, the lamB protein. Parts of this protein protruding above the outside of the membrane are shown. The numbers correspond to the amino acids of lamB numbered from the N-terminus of the nature protein. The black squares represent schematically the sites of insertion of the sequence derived from VP1 in the lamB protein. The designations 352C3, 264C3, 73C3 and 178C3 correspond to the four clones which were selected (AJC 352, AJC 264, AJC 73, AJC 178).

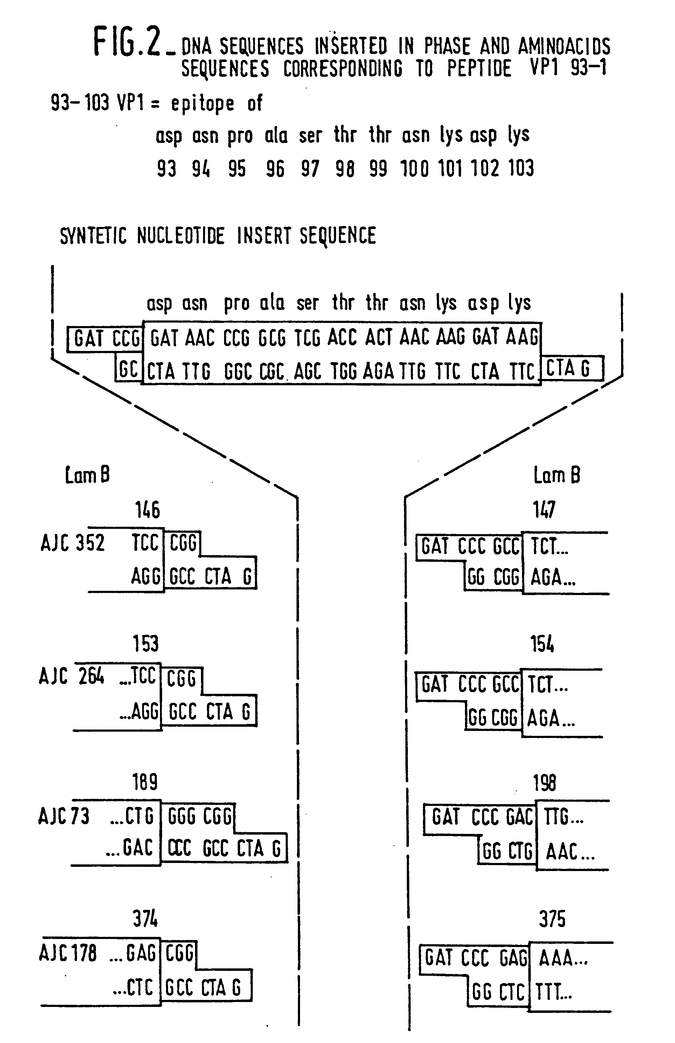

In Figure 2 are shown :

- the amino acid sequence of the epitope 93-103 C3, in the upper part of the Figure,

- the component parts of the adaptor which were prepared by synthesis and inserted at the sites 146, 153, 189 and 374 of the lamB protein, after cleavage with the BamHI enzyme and
- in the centre of the Figure is shown the nucleotide sequence prepared by synthesis and containing the epitope 93-103 C3 surrounded by cohesive ends with BamHI specificity.

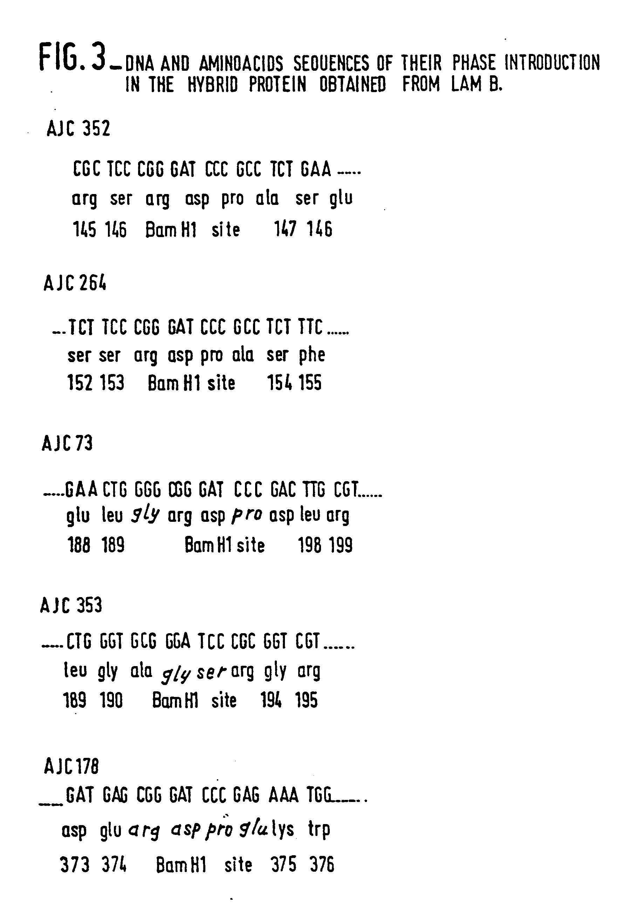

Figure 3 presents both the nucleotide and peptide sequences of the region of the three plasmids referred to above, but before the introduction at those sites of the insertion sequences containing the epitope.

## TABLE I

### CHARACTERISTICS OF THE LAMB - VP1 HYBRIDS

| Name of the mutants | AJC352 VP1 | AJC352 | AJC264 VP1 | AJC264 | AJC73 VP1 | AJC73 | AJC178 VP1 | AJC178 | AC1 |
|---|---|---|---|---|---|---|---|---|---|
| Site of insertion of peptide VP1 | 146 | - | 153 | - | 189 | - | 374 | - | - |
| Amino acid content of protein (number) | 438 | 425 | 438 | 425 | 431 | 418 | 438 | 425 | 421 |
| Immuno-blot with an anti-lamB antiserum (denaturing conditions) | >47K | 47K | >47K | 47K | <47K | <47K | >47K | <47K | 47K |
| Immunoprecipitation with an anti-lamB antiserum (non-denaturing conditions) | + | + | + | + | + | - | + | + | + |
| Immuno-blot with an anti-VP1 antiserum (denaturing conditions) | >47K | - | >47K | - | - | - | >47K | - | - |
| Immunoprecipitation with anti-VP1 antiserum | +- | - | + | - | + | - | + | - | - |
| with anti-C3 antiserum (non-denaturing conditions) | - | - | +++ | - | - | - | + | - | - |
| Detection in situ with anti-VP1 antiserum | - | - | + | - | - | - | - | - | - |
| with anti-C3 antiserum | - | - | + | - | - | - | + | - | - |
| Sensitivity to IPTG | R | R | R | R | S | S | R | R | R |
| Sensitivity to phages  -h+ | R | R | R | I | R | R | R | R | S |
| -ho | R | R | S | S | R | R | R | I | S |
| -lh+ | R | S | S | S | R | R | S | S | S |
| h10 | R | R | R | R | R | R | R | R | S |
| McConkey colour dextrins | red | red | red | red | red | pink | red | red | red |

Meaning of letters relating to
- sensitivity to phage :
  - R = resistant
  - S = sensitive

- sensitivity to IPTG :
  - R = non-toxic
  - S = toxic (see text)

It will immediately be apparent to the man skilled in the art that the type of manipulation just described is applicable to any other type of protein, on the one hand, and to any other type of insertion sequence, on the other. In particular, it will be seen that any epitope other than that derived from the VP1 protein of poliovirus can be used, and, conversely, that the insertion of the chosen epitope into any other type of protein can be contemplated. Needless to say that the protein activity as the "carrier protein" should nevertheless be of a sufficient size in order to be able to resume a configuration such as to exhibit the properties sought for said protein, despite the insertion of the foreign epitope at the appropriate place therein.

Obviously, such considerations are within the normal skills of the man skilled in the art and, where the size of the carrier protein relative to that of the epitope may possibly be small to determine whether the process of the invention as defined more broadly hereafter would be operable or not.

As a consequence, the invention is applicable to the production of any nucleic acid, in particular a vector, containing initially a nucleotide sequence coding for a specific protein under the control of an appropriate promoter. It is also evident that the insertion technique which has just been presented relating to the incorporation of the epitope of the VP1 protein of the poliovirus into a plasmid containing a sequence coding for the protein lamB, is applicable to the modification, by the insertion of a sequence coding for an epitope, of any other nucleic acid containing initially a nucleotide sequence coding for another defined protein, should it be desirable to produce a hybrid protein capable of expressing at least that biological property of that another protein one wishes to preserve together with the properties characteristic of the epitope.

The process according to the invention can thus be general by defined as follows. The process according to the invention for the modification of nucleic acids containing initially a nucleotide sequence coding for a specific protein, by the insertion into this nucleotide sequence of a distinct insertable nucleotide fragment coding for a peptide carrying an epitope under conditions which, when at least one of these modified nucleic acids has been incorporated into competent cells, lead to the production of a hybrid protein capable of exhibiting simultaneously at least those biological properties of the protein which are sought in a selected environment on the one hand , and the properties characteristic of the epitope, on the other hand,is characterised by the following sequence of operations :

- opening or cleaving of these nucleic acids at sites thereof to produce a multiplicity of linearised nucleic acids,
- reconstituting by in vitro recombination of said multiplicity of linearised nucleic acids and the ligation, simultaneously or subsequently, of the said insertable fragment within at least some of these nucleic acids in order to obtain a series of reconstituted nucleic acids which include nucleic acids containing said fragment, inserted at distinct sites in these nucleic acids,
- transforming competent cells with the said series of nucleic acids and the culture of the competent cells thus transformed,
- detecting and isolating those colonies which have been caused to express simultaneously the biological properties sought of the protein in said appropriate environment on the one hand, and of the peptide carrying said epitope on the other hand.
- if appropriate, recovering from the colonies thus isolated of the corresponding modified nucleic acids containing the nucleotide sequences coding for the protein, as modified though by the insertion therein at the appropriate sites of the above-mentioned insertable nucleotide fragment.

Seen in another light, the invention may be considered as consisting of a process for the insertion of a peptide containing a specific epitope in a protein exhibiting specific biological properties and which is capable of being produced in competent cells transformed by a nucleic acid containing initially a nucleotide sequence coding for the said protein without incurring the loss of essential biological properties of both the protein and the peptide in the selected environment, this procedure being characterised by the following sequence of steps :

- opening or cleaving these nucleic acids at sites which are distinct from one nucleic acid to another, in order to produce a multiplicity of linearised nucleic acids or fragments of nucleic acids,
- reconstituting by in vitro recombination of said multiplicity of linearised nucleic acids and the ligation, simultaneously or subsequently, of an insertable fragment coding for the above mentioned peptide into at least some of these reconstituted nucleic acids in order to obtain a series of reconstituted nucleic acids which include nucleic acids containing said insertable fragment, inserted at distinct sites in these nucleic acids,
- transforming competent cells with the said series and culturing of the competent cells thus transformed,
- detecting and isolating those colonies which have been caused to express simultaneously the biological properties sought of the protein and of the peptide in said selected environment,
- recovering the colonies thus isolated containing or producing the above-mentioned protein modified by a peptide containing the above-mentioned epitope, inserted at sites in the original protein which are compatible with the preservation of essential properties which the protein originally possessed (or the manifestation of said essential properties in said selected environment) simultaneously, with the acquisition of the properties characteristic of the epitope, and

- if appropriate recovering the modified protein.

Although preferred, the non-specific endonuclease can be replaced by a restriction enzyme, even a "cocktail" of restriction enzymes. The cleavages - in the case of a partial digestion - are then no longer quite at random along the nucleotide chain. However, they can, if necessary, be made at a sufficient number of sites in the nucleotide chain of the nucleic acid to lead to insertions, less perfectly to be sure, in some cases at a number of sites sufficient to enable hybrid sequences to be selected which provide final results similar to those obtained as a result of the process alternatives defined above.

The opening or cleavage of the above-mentioned nucleic acids at sites which are distinct from a particular nucleic acid to another is advantageously carried out by at least one non-specific endonuclease or by any other appropriate means (ultra-sonics, enzymes, shearing forces, etc..) which produce openings or cleavages at sites distributed at random in these nucleic acids.

The nucleic acids used in the process according to the invention are, preferably, circular DNAs, plasmid vectors, circular phages or cosmids. The above-mentioned "reconstitution" of these nucleic acids thus implies their recircularisation. The invention is, however, not limited to the use of such nucleic acids. It can also be applied to nucleic acids which meet the same criteria but which are not circularised. In the latter case they may be cleaved into fragments by an endonuclease at distinct sites in the nucleotide sequence, whereby the recircularisation step mentioned previously is replaced by recombination leading to the reconstitution of a series of modified nucleic acids, as stated above, among which nucleic acids containing the insertable fragment, inserted at distinct sites in the respective chains of said nucleic acid. Such nucleic acids are, for example, constituted by certain phages which, by nature, are not circular and which can be modified by a nucleotide sequence coding for the above-mentioned peptide, especially in one of their non-essential regions.

At this point it may be noted that, as far as the "biological properties" mentioned earlier are concerned, they are not limited to those which play a role in immunological reactions. Other properties may also be implicated : capacity for reactions with related molecules, activity towards a specific substrate, capacity of bacteria modified by the protein formed to grow in certain selective media, etc...

It will be clear to man skilled in the art that modifications can be made to the technique used, depending on the nature of the nucleotide constituents implicated in the production of a defined hybrid protein. Preferably, recourse is had to the recircularisation of linearised plasmids under the conditions mentioned above (if necessary, after repair of the ends of the plasmid by means of a polymerase to produce blunt ends), in order to facilitate the subsequent insertion of an insertion sequence coding for a peptide containing the chosen epitope. This method also ensures better control of the sequence studied. However, the use of such adaptors is not always necessary. The recircularisation of plasmids which have been linearised beforehand in a random manner and the ends of which have been repaired, can be performed directly in the presence of the sequence coding for the peptide to be inserted, which is there also provided with blunt ends, and of the appropriate ligase.

Alternatively and advantageously the sequence coding for the peptide to be inserted can be ended on both sides by a linker (or linkers) encoding restriction sites.

The invention is thus generally applicable to the production of other types of living vaccines, implying the use of any non-pathogenic bacterial strain which can be transformed by a vector containing a hybrid sequence such as that defined above and coding for a protein capable of being transported to the surface of the said cells under the conditions defined above.

The invention thus also relates to vaccine compositions formed with such "live vaccines" by combining them with a pharmaceutical vehicle appropriate for the in vivo administration of the vaccine, in particular by the oral or parenteral route. The bacteria which can be used as carriers are not limited to non-pathogenic E. coli strains.

In this respect, and only as an illustration, mention will be made of non-pathogenic and non-invasive strains of Salmonella typhi which can be used as vaccines, for example Ty21 A mutants for man, or non-pathogenic and non-invasive strains of Salmonella typhimurium for example aro B⁻ mutants for animals. As examples of coding sequences for other carrier proteins, the proteins known by the designations OmpF, OmpC, OmpA, BtuB, PhoE, etc.; will be cited.

More especially, the invention relates to the particularly advantageous vector which contains a squence coding for the lamB protein under the control of a promoter which enables the entire sequence to be transcribed and translated in a strain of E. coli, transformed beforehand by this vector, which is characterised by the insertion of a nucleotidic sequence encoding a determined epitope at an appropriate site of said sequence coding for the lamB protein, said appropriate place being such as to provide for the insertion of the determined epitope at a site located in the region coding for the amino acids 140 to 160 of the mature lamB protein or in the region coding for the amino acids 370 to 380. Preferred sites for insertion into the lamB protein are located at the level of amino acids in positions 153 and 374, respectively.

It may be pointed out that the inserted sequence usually consists of from 4 to 44 eg. from 4 to 35 amino acids, more particularly from 4 to 27, although these numbers are not to be taken in any way as limitative.

Consequently, the invention also relates to E. coli bacterial strains resulting from the transformation of E. coli by the vector defined above, these E. coli bacterial strains bearing on their external surface modified receptors for the phage which contain, exposed at the surface, more especially a peptide sequence comprising a characteristic and distinct epitope inserted in an insertion site located between two amino acids located between amino residues at positions 150 to 160, and, in particular, at position 153 of the lamB protein.

The invention is also applicable to the synthesis of hybrid proteins containing several epitopes.

Advantageously, the cell system and the vectors used are chosen such that the desired hybrid protein finally obtained is excreted into the culture medium. A case in point would be the insertion of a DNA sequence coding for a peptide containing a selected epitope into the nucleic acid of the genome of the hepatitis virus B which contains the pre-S and S regions of this virus, and the transformation of a susceptible eucaryotic host cell which is then able to excrete the HBsAg antigen into the culture medium.

The cell system can itself be constituted by eucaryotic cells which have been transformed by a vector containing a part of said pre-S and S regions and allowing for there respective expressions. Here, too, the invention must make it possible to define that part of the sequence containing the pre-S and S regions mentioned in which can be inserted the sequence coding for the selected epitope can be inserted, while thereby preserving the immunogenic properties of the HBs antigen and simultaneously ensurring the appropriately exposure of the epitope on the surface of the HBs antigen particles, in order that its immunogenic properties be expressed too when the hybrid antigen expected to be formed is administered in vivo.

In a general sense, the invention thus also provides a technique for analysing the sites on a protein at which a specific epitope can be introduced. The procedure according to the invention thus provides new means to study the topology and structure of specific proteins by the insertion of suitable sequences at different sites in these proteins and to evaluate the constraints which are exerted in the region of the protein at which the insertions are performed. Having defined such a region, it is also possible to verify whether peptides larger than those used for the insertion could be inserted in that particular region of the protein without perturbing those properties of the protein regarded as essential and the expression, if desired, of the biological properties intrinsic to the larger peptides.

Conversely, the procedure according to the invention is applicable to the identification of those regions in a defined protein which contain characteristic epitopes. This alternative of the process according to the invention thus includes steps which consist of fragmenting a DNA sequence coding for the protein under study or synthetizing chemically the fragments of the DNA sequences and of inserting the different fragments in a receptor protein in a region thereof previously identified as enabling appropriate exposure of the peptides encoded by DNA fragments inserted therein, whereby the expression of the different fragments can be studied after the hybrid sequence has been introduced into competent bacteria and caused to be expressed therein. This procedure will enable those regions of the protein under study to be identified which are recognized more strongly by antibodies produced against the whole protein. In particular, one or several epitopes of the protein under study can be identified by this approach (for example, by insertion at the site 153 of the lamB gene described above) and it also enables clones which express them to be prepared. By using a specific antiserum to the protein under study, it is possible to look for, and indeed isolate, colonies which bear an epitope. For example, this approach is applicable to the search for the epitopes of a viral protein using the serum of a patient.

The invention can be involved in numerous embodiments. The possibility of associating an epitope with a protein some properties of which must be preserved can be used for the purpose of conjugating in the hybrid protein the effects of both the original protein and inserted epitope. The resulting hybrid protein is a new product having specific properties different from the own properties of each component.

The interactions between the two elements of the hybrid protein can be used for example in diagnostic assays in vitro, which involve reactions of the antigen-antibody type between the epitope contained in the hybrid protein and an antibody directed against this epitope.

The invention is of particular interest when applicable to the production of a hybrid protein for very sensitive diagnostic assays, when the completion of the immunological reaction entailing the production of an immunological complex between the antibody against the epitope and the hybrid protein carrying said epitope also entails a modification of the properties of the carrier protein in the complex as formed, this modification being easily revealable.

10

A hybrid protein useful for this purpose comprises an enzyme containing an epitope inserted in such a location of the peptidic chain of the enzyme

- that the epitope is exposed such that, when said hybrid protein is contacted immunologically with antibodies against the epitope, a complex is formed between said hybrid protein and said antibodies,
- that the enzymatic activity of said enzyme is preserved in the hybrid protein in the non complexed state,
- that, when said complex is formed, the latter complex is devoid of said enzymmatic activity.

An alternative hybrid protein useful for diagnostic assays comprises an enzyme containing an epitope inserted in such a location

- that the enzymatic activity of said enzyme is inhibited in the hybrid protein in the non complexed state,
- that the epitope is exposed in such a way that when said hybrid protein is contacted immunologically with antibodies against the epitope, a complex is formed between said hybrid protein and said antibodies,
- that when said complex is formed, the latter complex has an enzymatic activity. For instance this effect can be obtained when the carrier protein is an enzyme (such as alkaline phosphatase, peroxidase or ß-galactosidase) and when the site of insertion of the epitope in the carrier protein is such that the enzymatic properties, e.g. the capacity to hydrolyse a substrate are preserved in the absence of antibodies against the inserted epitope, yet inhibited in the complex formed between the hybrid protein and the antibody.

A reagent is then obtained which is particularly easy to use. The diagnostic assay using such agent then comprises the steps of :

- contacting the said hybrid protein with said biological sample under conditions which enable the immunological reaction entailing the production of an immunological complex between the antibodies against the epitope and the hybrid protein carrying the epitope
- contacting the reaction product of said immunological reaction with the substrate hydrolyzable by said enzyme such as to enable the possible hydrolysis of said substrate and
- detecting the hydrolysis if any of the substrate by physical or chemical means, or with a naked eye.

Such reaction is particularly easy to achieve when the hydrolysis involves a reaction which can be detected by colorimetric or spectrophotometric equipment. This test can then be achieved in a homogeneous phase, particularly when the biological sample is a liquid.

Such an hybrid protein can be manufactured by the process of the invention, as defined above, but the operation of detection and isolation of the competent colonies will have to meet an additional condition. As a matter of fact it is recalled that the process according to the invention comprised a step of detection and isolation of those of the colonies whose expression products express or have been caused to express simultaneously the biological properties of the protein sought to be preserved (here, the capacity of this protein to hydrolyse a dertermined substrate) and of the polypeptide or epitope inserted in the enzyme (particularly the capacity of this epitope to be recognised by the antibodies).

For the purpose of obtaining a hybrid enzyme suitable for use in the diagnostic process mentioned above, it will be necessary to further detect those of the colonies which produce an hybrid enzyme satisfying the conditions which have been recalled for example e.g. whose enzymatic activity is preserved in the absence of, and inhibited when reacted with an antibody which recognises specially the epitope.

Conversely the invention is also applicable for the production of an hybrid enzyme containing an epitope, such that the desired capacity of the enzyme to express its natural property consisting in hydrolysing a specific substrate is inhibited in the absence of, yet is restored in presence of an antibody directed against the epitope.

Thus, one will also observe that the above expressed conditions concerning the capacity of the hybrid protein to "exhibit the properties sought of the initial protein in the appropriate environment" may have to take into account additional parameters necessary for causing the properties sought to be expressed. In the instant case the suitable environment would involve the additional presence of the antibodies against the epitope, in order to enable the properties sought of the initial protein - e.g. its enzymatic properties - to be expressed by the hybrid protein.

Such a hybrid protein is then suitable for use in diagnostic assays of the type considered above, except for the reversal of the detected phenomena. Thus, the hybrid enzyme would have the property of being unable to express the natural property of the not-modified enzyme in the presence of the corresponding substrate, whereas the enzymatic activity would be restored after contacting of the hybrid protein with the antibody directed against the epitope.

11

The combined use of the interesting properties of the protein and of the properties of the epitope, in the hybrid protein obtained by the process according to the invention is particularly advantageous in the case of application to diagnostic. Indeed this application permits to achieve an immuno-enzymatic essay in homogeneous phase without separating the constituents.

The hybrid protein according to the invention is also useful for any form other than the form of diagnosis disclosed hereabove of the possible content of antibodies against the epitope of the hybrid protein in a biological sample.

An indirect method for diagnosing the possible antibodies comprises for instance :
- adding to said biological sample a determined amount of labeled antibodies specifically recognizing the epitope,
- contacting the mixture obtained with a determined amount of hybrid protein under conditions enabling the production of immunological complexes between both the labelled antibodies and the antibodies of the biological sample if any, on the one hand, and said hybrid protein on the other hand, whereby said determined amount of labelled antibodies and hybrid protein respectively are adjusted such as to provide for a competition between un labeled antibodies if any and the labeled antibodies and measuring the contents of antibodies in said biological sample as a function of the relative proportion of the labelled antibodies which have been found to be retained in the immunological complex under equilibrium conditions.

Another indirect method for diagnosing in vitro the possible content of antibodies against the epitope of the hybrid protein comprises :
- contacting the biological sample to be assayed with a known quantity of the hybrid protein bound to a solid support surface in conditions authorizing the production of a immunological complex between the epitope of the hybrid protein and the antibody to be detected, if any, and
- contacting the immunological reaction product obtained with a labeled reagent specifically recognizing the immunoglobulins of immunological reaction product. By way of example said reagent consists of antibodies directed against immunoglobulins of a bacterial protein A, particularly of staphyloccocus aureus, and
- detecting the labelled insoluble three-part immunological complex
- optionally, measuring the amount of three-part-complex formed.

The invention relates also to kits for diagnosing in vitro the possible content of antibodies against the epitope of the hybrid protein in a biological sample.

One of the preferred kits according to the invention comprises :
- a determined amount of the hybrid protein according to the invention, this hybrid protein being capable of producing immunological complexes with the antibodies of the biological sample, if any and/or with the labeled antibodies,
- a determined amount of labeled antibodies specifically recognizing the epitope of said hybrid protein,
- optionally a reference curve, permitting the measuring by means of comparison, of the amount of antibodies contained in the biological sample as a function of the amount of complex formed between the labeled antibodies and the hybrid protein,
- reagents permitting the detection of the immunological complexes containing the antibodies and the hybrid protein, e.g. the substrate of a determined enzyme, when said label comprises said determined enzyme.
- an appropriate medium which enables the production of the various immunological complexes.

Another preferred kit for the diagnosis, according to the invention is a kit which comprises :
- a determined amount of the hybrid protein according to the invention, bound to a support
- a determined amount of antibodies directed against the epitope of said epitope,
- media authorising the production of the immunological reactions between the antibodies against the epitope of the hybrid protein on the one hand, and between immunoglobulins and the labelled reagent on the other hand,
- a labelled reagent for detecting a three part immunological complex formed between support-bound-hybrid protein and the antibodies against the epitope of said hybrid protein, this reagent specifically recognizing the immunoglobulins of immunological reaction product. By way of example said reagent consists of antibodies directed against immunoglobulins of a bacterial protein A, particularly of staphyloccocus aureus.

Preferably the antibodies used in the methods and kits discloses above are monoclonal antibodies.

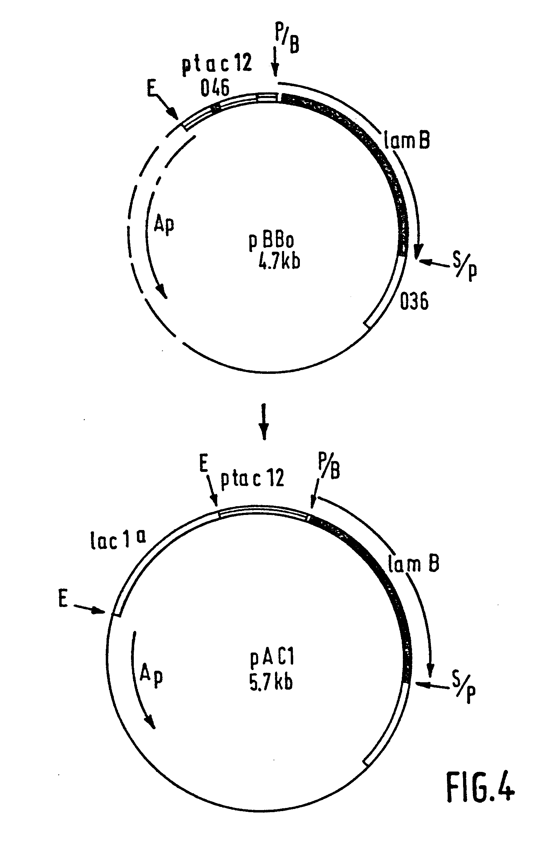

Figure 4 shows schematically the structures of two plasmids which were used to implement the invention. In particular, they consist of plasmid derivatives of pBR322 in which a sequence of lamB was inserted under the control of the promoter TAC 12. The letters P/B relate to the joining performed at the former pvuII/BamHI Site of the two fragments cited above (proximal part of lamB) and the letter S/P to joining performed at the former StuI/PvuII Site of lamB (distal part) and pBR322. The plasmid pACI differs from plasmid pBBo by the presence in the former of a lacI^q sequence containing the repressor of the lactose operon.

REFERENCES

- Amann, E., Brosius, J. et Ptashne M. (1983) Gene, 25, 167-178.
- Bagdasarian, M.M., Amann, E., Lurtz, R., Ruckert, B. et Bagdadsarian, M. (1983) Gene, 26, 273-282.
- Charbit, A., Clément, J. M. et Hofnung, M. (1984) J. Mol. Biol., 175, 395-401.
- Charbit, A. et Hofnung, M. (1985) Journal of Virology, 53, 667-671.
- Clément, J. M. et Hofnung, M. (1981) Cell, 27, 507-514.
- Guesdon, J. L., Bouges-Bocquet, B., Débarbouillé,. M. et Hofnung, M. (1985) J. Immunolo. Methods, sous presse.
- Heffron, F. So, M. et Mc Carthy,B.J. (1978) P.N.A.S. (USA), 75, 6012-6016.
- Hengge, R. et Boos, W. (1983) Biochim. Biophy. Acta 737, 443-478.
- Hogle, J.M., Chow,M. et Filman, D.J. (1985) Science, 229, 1358-1365.
- Lathe, R., Kierry, M.P., Skory, S. et Lecocq, J.P. (1984) DNA, 3 N° 2, 173-182.
- Van der Werf, S., Wychowski, C. Bruneau, P., Blondel, B., Crainic, R., Horodniceanu, F. et Girard, M. (1983). P.N.A.S. (USA) 5080-5084.
- Wychowski, C., Van der Werf, S., Siffert, O, Crainic, R., Bruneau, P. et Girard, M. (1983) Embo Journal, 2 N° 11, 2019-2024.

**Claims**

1. A process for the modification of nucleic acids containing initially a nucleotide sequence coding for a specific protein by the insertion into this nucleotide sequence of a distinct insertable nucleotide fragment coding for a peptide bearing an epitope under conditions which, when at least one of these modified nucleic acids has been incorporated into competent cells, leads to the production of a hybrid protein capable of exhibiting simultaneously at least those biological properties of the protein which are sought in a selected environment on the one hand, and the properties characteristic of the epitope, on the other hand, is characterised by the following sequence of steps :
   - opening or cleaving of these nucleic acids at sites thereof to produce a multiplicity of linearised nucleic acids,
   - reconstituting by in vitro recombination of said multiplicity of linearised nucleic acids and the ligation, simultaneously or subsequently, of the said insertable fragment within at least some of these nucleic acids in order to obtain a series of reconstituted nucleic acids which include nucleic acids containing said fragment, inserted at distinct sites in these nucleic acids,
   - transforming of competent cells with the said series of nucleic acids and the culture of the competent cells thus transformed,
   - detecting and isolating those colonies which have been caused to express simultaneously the biological properties sought of the protein in said appropriate environment on the one hand, and of the peptide carrying said epitope, in the other hand,
   - if appropriate, recovering from the colonies thus isolated of the corresponding modified nucleic acids containing the nucleotide sequences coding for the protein, as modified though by the insertion therein at the appropriate sites of the above-mentioned insertable nucleotide fragment.

2. A process for the insertion of a peptide containing a specific epitope in a protein exhibiting specific biological properties and which is capable of being produced in competent cells transformed by a nucleic acid containing initially a nucleotide sequence coding for the said protein without incurring the loss of essential biological properties of both the protein and the peptide in the selected environment, characterised by the following sequence of steps :
   - opening or cleaving these nucleic acids at sites which are distinct from one nucleic acid to another, in order to reproduce a multiplicity of linearised nucleic acids or fragments of nucleic acids,
   - reconstituting by in vitro recombination of said multiplicity of linearised nucleic acids and the ligation, simultaneously or subsequently, of an insertable fragment coding for the above-mentioned peptide into at least some of these reconstituted nucleic acids in order to obtain a series of nucleic acids which include nucleic acids containing said insertable fragment, inserted at distinct sites in these nucleic acids,

- transforming competent cells with the said series and the culturing the competent cells thus transformed,

- detecting and isolating those colonies which have been caused to express simultaneously the biological properties sought of the protein and the peptide in said selected environment.

- recovering the colonies thus isolated containing or producing the above-mentioned protein modified by a peptide containing the above-mentioned epitope, inserted at sites in the original protein which are compatible with the preservation of the essential properties which the protein originally possessed (or the manifestation of said essential properties in said selected environment) and, simultaneously, with the acquisition of the properties characteristic of the epitope, and

- if appropriate, recovering the modified protein.

3. Process according to claim 1 characterised in that the cleavages of the above-mentioned nucleic acids at distinct sites are performed at sites distributed at random along the nucleic acids.

4. Process according to claim 2 characterised in that the cleavages of the above-mentioned nucleic acids at distinct sites are performed at sites distributed at random along the nucleic acids

5. Process according to the claim 3, characterised in that the cleavage of the nucleic acids at sites which are distinct for each nucleic acid is carried out by means of at least one non-specific endonuclease.

6. Process according to the claim 1, characterised in that the nucleic acids to be modified are circular, and that the cleavage of these nucleic acids is carried out so as to produce a linearisation of the nucleic acids. Their subsequent in vitro recombination leading to the production of a set of recircularised nucleic acids which include nucleic acids containing the fragment inserted at distinct sites.

7. Process according to the claim 1, characterised in that the circular nucleic acid is a vector, that the recombination is carried out in the presence of an adaptor containing a specific restriction site, in that the fragment coding for the peptide containing the epitope has cohesive ends characteristic of this restriction site and in that the fragment is subsequently inserted into the restriction sites introduced into the modified vectors by the adaptor and recombined.

8. Process according to the claims 6, characterised in that the circular nucleic acid is a vector, that the recombination is carried out in the presence of an adaptor containing a specific restriction site, in that the fragment coding for the peptide containing the epitope has cohesive ends characteristic of this restriction site and in that the fragment is subsequently inserted into the restriction sites introduced into the modified vectors by the adaptor and recombined.

9. Process according to the claim 1, characterised in that the protein encoded in the above-mentioned nucleotide sequence is a protein normally transported to the external surface of competent producer cells or excreted by them into the culture medium.

10. Process according to claim 9, characterised in that the nucleotide sequence coding for the specific protein is constituted by the lamB gene, or at least that part of it coding for the lamB protein and in that the competent cells used are those of a bacterial strain.

11. Process according to the claim 10, characterised in that the determination and the isolation of the above-mentioned colonies depends, on the one hand, on detecting the sensitivity of the transformed bacteria to phages using the λ receptor or their capacity to grow on dextrins and, on the other hand, on the recognition of the epitope by a monoclonal antibody which specifically recognizes it.

12. Vector containing a sequence coding for the lamB protein under the control of a promoter which allows the whole of this sequence to be transcribed and then translated in a strain of E. coli or another Gram-negative bacterium which has been previously transformed by the vector, characterised by the insertion of an nucleotidic sequence coding for a peptide sequence bearing a distinct epitope at a site situated in the region coding for the aminoacids from 140 to 160 and from 370 to 380 of the mature lamB protein.

13. Vector according to claim 12, characterised in that the sequence inserted contains 4 to 27 amino acids.

14. Vector according to claim 12, characterised in that the sequence inserted contains a nucleotide sequence coding for an epitope of poliovirus type I, in particular the peptide :
asp asn pro ala ser thr thr asn lys asp lys.

15. Strain of E. coli bacteria bearing on its external surface modified receptors for the phage λ containing, exposed at the surface, more particularly a peptide sequence containing a characteristic epitope inserted between two amino acids located between the positions 140 and 160 or 370 and 380 of the amino acid sequence, and more particularly at positions 153 or 374 of the lamB protein.

16. Immunogenic preparation directed against pathogenic antigens neutralizable by antibodies directed against a specific epitope, characterised by the combination of bacteria in accordance with claim 15, in which the epitope meets the condition specified above, with a pharmaceutical vehicle appropriate for the production of vaccines.

17. A hybrid protein comprising an enzyme containing an epitope inserted in such a location of the peptidic chain of the enzyme
    - that the epitope is exposed such that, when said hybrid protein is contacted immunologically with antibodies against the epitope, a complex is formed between said hybrid protein and said antibodies,
    - that the enzymatic activity of said enzyme is preserved in the hybrid protein in the non complexed state,
    - that, when said complex is formed, the latter complex is devoid of said enzymatic activity.

18. A hybrid protein comprising an enzyme containing an epitope inserted therein wherein the epitope is inserted in such a location
    - that the epitope is exposed in such a way that when said hybrid protein is contacted immunologically with antibodies against the epitope, a complex is formed between said hybrid protein and said antibodies,
    - that the enzymatic activity of said enzyme is inhibited in the hybrid protein the non complexed state,
    - that, when said complex is formed, the latter complex has an enzymatic activity.

19. Method for diagnosing the possible contents of antibodies in a biological sample comprising :
    - contacting the hybrid protein of claim 17 with said biological sample under conditions which enable the immunological reaction entailing the production of an immunological complex between the antibodies against the epitope and the hybrid protein carrying the epitope
    - contacting the reaction product of said immunological reaction with the substrate hydrolyzable by said enzyme such as to enable the possible hydrolysis of said substrate
    - detecting the hydrolysis, if any, of the substrate by physical or chemical means.

20. Method for diagnosing the possible contents of antibodies in a biological sample comprising :
    - contacting the hybrid protein of claim 18 with said biological sample under conditions which enable the immunological reaction entailing the production of an immunological complex between the antibodies against the epitope and the hybrid protein carrying the epitope
    - contacting the reaction product of said immunological reaction with the substrate hydrolyzable by said enzyme such as to enable the possible hydrolysis of said substrate
    - detecting the hydrolysis, if any, of the substrate by physical or chemical means.

**Patentansprüche**

1. Verfahren zur Modifikation von Nucleinsäuren, die anfangs eine ein spezifisches Protein codierende Nucleotidsequenz enthalten, durch die Insertion eines bestimmten insertierbaren Nucleotidfragments, welches ein ein Epitop tragendes Peptid codiert, unter Bedingungen, die, wenn mindestens eine dieser modifizierten Nucleinsäuren in kompetente Zellen eingebaut ist, zu der Produktion eines Hybridproteins führt, das fähig ist, gleichzeitig mindestens einerseits die biologischen Eigenschaften des Proteins zu zeigen, die in einer gewählten Umgebung erwünscht sind, und andererseits die Eigenschaften, die

EP 0 242 243 B1

charakteristisch für das Epitop sind, welches durch die folgende Reihenfolge von Schritten charakterisiert ist:

Öffnen oder Spalten dieser Nucleinsäuren an bestimmten Stellen, um eine Vielzahl von linearisierten Nucleinsäuren zu erhalten,

Rekonstitution durch in vitro-Rekombination der Vielzahl von linearisierten Nucleinsäuren und gleichzeitige oder aufeinanderfolgende Ligierung des insertierbaren Fragments innerhalb zumindest einiger dieser Nucleinsäuren, um eine Reihe von rekonstituierten Nucleinsäuren zu erhalten, die Nucleinsäuren umfassen, welche dieses Fragment an bestimmten Stellen in diesen Nucleinsäuren insertiert enthalten,

Transformieren von kompetenten Zellen mit dieser Reihe von Nucleinsäuren und Züchtung der so transformierten kompetenten Zellen,

Nachweis und Isolierung von Kolonien, die veranlaßt wurden, gleichzeitig die biologischen Eigenschaften zu exprimieren, die einerseits von dem Protein in der geeigneten Umgebung erwünscht werden und andererseits die des das Epitop tragenden Peptids sind,

gegebenenfalls Gewinnung aus den so isolierten Kolonien der entsprechenden modifizierten Nucleinsäuren, die die das Protein codierenden Nucleotidsequenzen enthalten, die jedoch durch die Insertion des vorstehend genannten insertierbaren Nucleotidfragments an geeigneten Stellen modifiziert sind.

2. Verfahren zur Insertion eines ein spezifisches Epitop enthaltenden Peptids in ein Protein, das spezifische biologische Eigenschaften aufweist und das in kompetenten Zellen produziert werden kann, die durch eine Nucleinsäure transformiert wurden, welche anfangs eine das Protein codierende Nucleotidsequenz enthielten, ohne daß wesentliche biologische Eigenschaften sowohl des Proteins als auch des Peptids in der gewählten Umgebung verloren gehen, gekennzeichnet durch die folgende Reihenfolge von Schritten:

Öffnen oder Spalten dieser Nucleinsäuren an Stellen, die unterschiedlich sind von einer Nucleinsäure zur anderen, um eine Vielzahl von linearisierten Nucleinsäuren oder Fragmenten von Nucleinsäuren zu reproduzieren,

Rekonstitution durch in vitro-Rekombination der Vielzahl von linearisierten Nucleinsäuren und gleichzeitige oder aufeinanderfolgende Ligierung eines das vorstehend erwähnte Peptid codierenden Fragments in mindestens einige dieser rekonstituierten Nucleinsäuren, um eine Reihe von Nucleinsäuren zu erhalten, die Nucleinsäuren umfassen, welche das insertierbare Fragment enthalten, wobei es an unterschiedlichen Stellen in diesen Nucleinsäuren insertiert ist,

Transformieren kompetenter Zellen mit dieser Reihe und Züchtung der so transformierten kompetenten Zellen,

Nachweis und Isolierung der Kolonien, die veranlaßt wurden, gleichzeitig die biologischen Eigenschaften zu exprimieren, die von dem Protein und dem Peptid in der gewählten Umgebung erwartet werden,

Gewinnung der so isolierten Kolonien, die das vorstehend genannte Protein enthalten oder produzieren, welches durch ein Peptid modifiziert ist, das das vorstehend genannte Epitop an verschiedenen Stellen im Orginalprotein insertiert enthält, die kompatibel sind mit der Aufrechterhaltung wesentlicher Eigenschaften, die das Protein ursprünglich besaß (oder der Manifestation dieser wesentlichen Eigenschaften in der ausgewählten Umgebung) und gleichzeitig mit der Erlangung der für das Epitop charakteristischen Eigenschaften, und

gegebenenfalls Gewinnung des modifizierten Proteins.

3. Verfahren nach Anspruch 1, dadurch gekennzeichnet, daß die Spaltungen der vorstehend genannten Nucleinsäuren an unterschiedlichen Stellen an Stellen durchgeführt werden, die zufällig entlang der Nucleinsäuren verteilt sind.

4. Verfahren nach Anspruch 2, dadurch gekennzeichnet, daß die Spaltungen der vorstehend genannten Nucleinsäuren an unterschiedlichen Stellen an Stellen durchgeführt werden, die zufällig entlang der Nucleinsäuren verteilt sind.

5. Verfahren nach Anspruch 3, dadurch gekennzeichnet, daß die Spaltung der Nucleinsäuren an Stellen, die unterschiedlich für jede Nucleinsäure sind, mit Hilfe von mindestens einer nicht-spezifischen Endonuclease ausgeführt werden.

6. Verfahren nach Anspruch 1, dadurch gekennzeichnet, daß die zu modifizierenden Nucleinsäuren zirkulär sind und daß die Spaltung dieser Nucleinsäuren so ausgeführt wird, daß eine Linearisierung der Nucleinsäuren erfolgt, wobei die nachfolgende in vitro-Rekombination zur Produktion eines Satzes von

16

rezirkularisierter Nucleinsäuren führt, welche Nucleinsäuren umfassen, die das Fragment an unterschiedlichen Stellen insertiert enthalten.

7. Verfahren nach Anspruch 1, dadurch gekennzeichnet, daß die zirkuläre Nucleinsäure ein Vektor ist, daß die Rekombination in Gegenwart eines Adaptors ausgeführt wird, der eine spezifische Restriktionsstelle enthält, daß das Fragment, welches das das Epitop tragende Peptid codiert, cohäsive Enden hat, die charakteristisch für diese Restriktionsstelle sind, und daß das Fragment anschließend in die Restriktionsstellen insertiert wird, welche in die modifizierten Vektoren durch den Adaptor eingeführt und rekombiniert wurden.

8. Verfahren nach Anspruch 6, dadurch gekennzeichnet, daß die zirkuläre Nucleinsäure ein Vektor ist, daß die Rekombination in Gegenwart eines eine spezifische Restriktionsstelle enthaltenden Adaptors ausgeführt wird, daß das Fragment, welches das das Epitop tragende Peptid codiert, cohäsive Enden hat, die charakteristisch für diese Restriktionsstelle sind, und daß das Fragment anschließend in die Restriktionsstellen insertiert wird, welche in die modifizierten Vektoren durch den Adaptor eingeführt und rekombiniert wurden.

9. Verfahren nach Anspruch 1, dadurch gekennzeichnet, daß das von der vorstehend genannten Nucleotidsequenz codierte Protein ein Protein ist, das normalerweise an die äußere Oberfläche von kompetenten Producerzellen transportiert wird oder von diesen in das Kulturmedium sekretiert wird.

10. Verfahren nach Anspruch 9, dadurch gekennzeichnet, daß die das spezifische Protein codierende Nucleotidsequenz aus dem lamB-Gen besteht oder mindestens aus dem Teil, der das lamB-Protein codiert, und daß die verwendeten kompetenten Zellen von einem Bakterienstamm stammen.

11. Verfahren nach Anspruch 10, dadurch gekennzeichnet, daß die Bestimmung und Isolierung der vorstehend erwähnten Kolonien einerseits vom Nachweis der Sensitivität der transformierten Bakterien gegenüber Phagen, die den λ-Rezeptor verwenden oder ihrer Fähigkeit auf Dextrinen zu wachsen, abhängt, und andererseits von der Erkennung des Epitops durch einen monoclonalen Antikörper, der es spezifisch erkennt.

12. Vektor, enthaltend eine lamB-Protein-codierende Sequenz, unter der Kontrolle eines Promotors, der es möglich macht, daß die ganze Sequenz transkribiert und translatiert wird in einem E.coli-Stamm oder einem anderen gram-negativen Bakterium, das vorher mit dem Vektor transformiert wurde, gekennzeichnet durch die Insertion einer Nucleotidsequenz, die eine Peptidsequenz codiert, welche ein bestimmtes Epitop an einer in dem Bereich liegenden Stelle trägt, der die Aminosäuren 140 bis 160 und 370 bis 380 des reifen lamB-Proteins codiert.

13. Vektor nach Anspruch 12, dadurch gekennzeichnet, daß die insertierte Sequenz 4 bis 27 Aminosäuren enthält.

14. Vektor nach Anspruch 12, dadurch gekennzeichnet, daß die insertierte Sequenz eine Nucleotidsequenz enthält, die ein Epitop des Poliovirus Typ I codiert, insbesondere das Peptid:
asp asn pro ala ser thr thr asn lys asp lys.

15. E.coli-Bakterienstamm, der auf seiner äußeren Oberfläche modifizierte Rezeptoren für den Phagen λ trägt, die, ausgesetzt an der Oberfläche, insbesondere eine Peptid-Sequenz mit einem charakteristischen Epitop aufweisen, das zwischen zwei Aminosäuren insertiert ist, die zwischen den Positionen 140 und 160 oder 370 und 380 der Aminosäuresequenz lokalisiert sind, und insbesondere an den Positionen 153 oder 374 des lamB-Proteins.

16. Immunogenes Präparat, das gegen pathogene Antigene gerichtet ist, die durch Antikörper neutralisierbar sind, die gegen ein spezifisches Epitop gerichtet sind, gekennzeichnet durch die Kombination von Bakterien gemäß Anspruch 15, wobei das Epitop die vorstehend genannten Bedingungen erfüllt, mit einem pharmazeutischen Träger, der für die Produktion von Impfstoffen geeignet ist.

17. Hybridprotein, umfassend ein Enzym, das ein Epitop enthält, welches so in einer Stelle der Peptidkette des Enzyms insertiert ist,

daß das Epitop so exponiert ist, daß, wenn das Hybridprotein immunologisch mit Antikörpern gegen das Epitop in Kontakt gebracht wird, ein Komplex zwischen dem Hybridprotein und den Antikörpern gebildet wird,
daß die enzymatische Aktivität des Enzyms in dem Hybridprotein im nicht-komplexierten Zustand erhalten ist,
daß, wenn der Komplex gebildet wird, der letztere Komplex ohne enzymatische Aktivität ist.

18. Hybridprotein, umfassend ein Enzym, welches ein darin insertiertes Epitop enthält, wobei das Epitop an einer solchen Stelle insertiert ist,
daß das Epitop so exponiert ist, daß, wenn das Hybridprotein immunologisch mit Antikörpern gegen das Epitop in Kontakt gebracht wird, ein Komplex zwischen dem Hybridprotein und den Antikörpern gebildet wird,
daß die enzymatische Aktivität des Enzyms in dem Hybridprotein im nicht-komplexierten Zustand gehemmt wird,
daß, wenn der Komplex gebildet wird, der letztere Komplex enzymatische Aktivität aufweist.

19. Verfahren zur Diagnose von möglichen Gehalten von Antikörpern in einer biologischen Probe, umfassend:
Inkontaktbringen des Hybridproteins nach Anspruch 17 mit der biologischen Probe unter Bedingungen, die die immunologische Reaktion ermöglichen, die zur Produktion eines immunologischen Komplexes zwischen den Antikörpern gegen das Epitop und dem das Epitop tragenden Hybridprotein führen,
Inkontaktbringen des Reaktionsprodukts der immunologischen Reaktion mit dem durch das Enzym hydrolysierbaren Substrat, so daß eine mögliche Hydrolyse des Substrats erfolgen kann,
Nachweis der Hydrolyse des Substrats, falls eine solche stattgefunden hat, durch physikalische oder chemische Mittel.

20. Verfahren zur Diagnose des möglichen Gehalts an Antikörpern in einer biologischen Probe, umfassend:
Inkontaktbringen des Hybridproteins nach Anspruch 18 mit der biologischen Probe unter Bedingungen, die die immunologische Reaktion ermöglichen, welche zur Produktion eines immunologischen Komplexes zwischen den Antikörpern gegen das Epitop und dem das Epitop tragenden Hybridproteion führen,
Inkontaktbringen des Reaktionsprodukts der immunologischen Reaktion mit dem durch das Enzym hydrolysierbaren Substrat, so daß die mögliche Hydrolyse des Substrats erfolgen kann,
Nachweis der Hydrolyse des Substrats, falls eine stattgefunden hat, durch physikalische oder chemische Mittel.

**Revendications**

1. Procédé de modification d'acides nucléiques, contenant initialement une séquence nucléotidique codant pour une protéine spécifique, par l'insertion dans cette séquence nucléotidique d'un fragment nucléotidique insérable distinct codant pour un peptide portant un épitope dans des conditions qui, lorsqu'au moins l'un de ces acides nucléiques modifiés a été incoporé dans des cellules compétentes, conduit à la production d'une protéine hybride susceptible de présenter simultanément au moins celles des propriétés biologiques de la protéine qui sont recherchées dans un environnement sélectionné, d'une part, et les propriétés caractéristiques de l'épitope, d'autre part, caractérisé par la séquence d'étapes suivante:
   - l'ouverture ou le clivage de ces acides nucléiques à des sites de ces derniers pour produire une multiplicité d'acides nucléiques linéarisés,
   - la reconstitution par recombinaison in vitro de ladite multiplicité d'acides nucléiques linéarisés et la ligature, simultanément ou ultérieurement, dudit fragment insérable à au moins quelques-uns de ces acides nucléiques de manière à obtenir une série d' acides nucléiques reconstitués comprenant les acides nucléiques contenant ledit fragment, inséré à des sites distincts dans ces acides nucléiques,
   - la transformation de cellules compétentes par ladite série d'acides nucléiques et la culture des cellules compétentes ainsi transformées,
   - la détection et l'isolement de celles des colonies qui ont été amenées à exprimer simultanément les propriétés biologiques recherchées pour la protéine dans ledit environnement approprié d'une part, et pour le peptide portant ledit épitope, d'autre part,

- éventuellement, la récupération, à partir des colonies ainsi isolées, des acides nucléiques modifiés correspondants contenant les séquences nucléotidiques codant pour la protéine, mais telles que modifiées par l'insertion dans celles-ci, aux sites appropriés du fragment nucléotidique insérable mentionné ci-dessus.

2. Procédé d'insertion d'un peptide contenant un épitope spécifique dans une protéine présentant des propriétés biologiques spécifiques et qui est susceptible d'être produite dans des cellules compétentes transformées par un acide nucléique contenant initialement une séquence nucléotidique codant pour ladite protéine sans entraîner la perte de propriétés biologiques essentielles aussi bien de la protéine que du peptide dans l'environnement sélectionné, caractérisé par la séquence d'étapes suivante:
   - l'ouverture ou le clivage de ces acides nucléiques à des sites distincts d'un acide nucléique à un autre, de manière à reproduire une multiplicité d'acides nucléiques linéarisés ou de fragments d'acides nucléiques,
   - la reconstitution par recombinaison in vitro de ladite multiplicité d'acides nucléiques linéarisés et la ligature, simultanément ou ultérieurement, d'un fragment insérable codant pour le peptide mentionné ci-dessus dans au moins quelques-uns de ces acides nucléiques reconstitués de manière à obtenir une série d'acides nucléiques dont les acides nucléiques contenant ledit fragment insérable, inséré à des sites distincts dans ces acides nucléiques,
   - la transformation de cellules compétentes par ladite série et la culture des cellules compétentes ainsi transformées,
   - la détection et l'isolement de celles des colonies qui ont été amenées à exprimer simultanément les propriétés biologiques recherchées pour la protéine et pour le peptide dans ledit environnement sélectionné,
   - la récupération des colonies ainsi isolées contenant ou produisant la protéine mentionnée ci-dessus modifiée par un peptide contenant l'épitope mentionné ci-dessus, inséré à des sites dans la protéine initiale, qui sont compatibles avec la conservation des propriétés essentielles que possédait la protéine initialement (ou la manifestation desdites propriétés essentielles dans ledit environnement sélectionné) et, simultanément, avec l'acquisition des propriétés caractéristiques de l'épitope, et
   - éventuellement, la récupération de la protéine modifiée.

3. Procédé selon la revendication 1, caractérisé en ce que les clivages des acides nucléiques mentionnés ci-dessus à des sites distincts sont effectués à des sites répartis au hasard le long des acides nucléiques.

4. Procédé selon la revendication 2, caractérisé en ce que les clivages desdits acides nucléiques mentionnés ci-dessus à des sites distincts sont effectués à des sites répartis au hasard le long des acides nucléiques.

5. Procédé selon la revendication 3, caractérisé en ce que le clivage des acides nucléiques à des sites qui sont distincts pour chaque acide nucléique est effectué à l'aide d'au moins une endonucléase non spécifique.

6. Procédé selon la revendication 1, caractérisé en ce que les acides nucléiques à modifier sont circulaires, et en ce que le clivage de ces acides nucléiques est effectué de manière à produire une linéarisation des acides nucléiques, leur recombinaison in vitro ultérieure conduisant à la production d'un ensemble d'acides nucléiques recircularisés dont les acides nucléiques contenant le fragment inséré à des sites distincts.

7. Procédé selon la revendication 1, caractérisé en ce que l'acide nucléique circulaire est un vecteur, en ce que la recombinaison est effectuée en présence d'un adaptateur contenant un site de restriction spécifique, en ce que le fragment codant pour le peptide contenant l'épitope possède des extrémités cohésives caractéristiques de ce site de restriction, et en ce que le fragment est ultérieurement inséré dans les sites de restriction introduits dans les vecteurs modifiés par l'adaptateur et recombiné.

8. Procédé selon la revendication 6, caractérisé en ce que l'acide nucléique circulaire est un vecteur, en ce que la recombinaison est effectuée en présence d'un adaptateur contenant un site de restriction spécifique, en ce que le fragment codant pour le peptide contenant l'épitope possède des extrémités cohésives caractéristiques de ce site de restriction et en ce que le fragment est ultérieurement inséré dans les sites de restrictions introduits dans les vecteurs modifiés par l'adaptateur et recombiné.

9. Procédé selon la revendication 1, caractérisé en ce que la protéine codée dans la séquence nucléotidique mentionnée ci-dessus est une protéine normalement transportée à la surface extérieure des cellules productrices compétentes ou excrétée par celles-ci dans le milieu de culture.

10. Procédé selon la revendication 9, caractérisé en ce que la séquence nucléotidique codant pour la protéine spécifique est constituée par le gène lamB, ou au moins par la partie de celui-ci codant pour la protéine lamB et en ce que les cellules compétentes mises en oeuvre sont celles d'une souche bactérienne.

11. Procédé selon la revendication 10, caractérisé en ce que la mise en évidence et l'isolement des colonies mentionnées ci-dessus dépend, d'une part, de la détection de la sensibilité aux phages des bactéries transformées à l'aide du récepteur λ ou leur capacité à croître sur les dextrines et, d'autre part, de la reconnaissance de l'épitope par un anticorps monoclonal le reconnaissant de manière spécifique.

12. Vecteur contenant une séquence codant pour la protéine lamB sous le contrôle d'un promoteur permettant à l'ensemble de cette séquence d'être transcrite et puis traduite dans une souche d'E. coli ou une autre bactérie Gram négatif précédemment transformée par le vecteur, caractérisé par l'insertion d'une séquence nucléotidique codant pour une séquence peptidique portant un épitope distinct à un site situé dans la région codant pour les acides aminés allant de 140 à 160 et de 370 à 380 de la protéine lamB mature.

13. Vecteur selon la revendication 12, caractérisé en ce que la séquence insérée contient 4 à 27 acides aminés.

14. Vecteur selon la revendication 12, caractérisé en ce que la séquence insérée contient une séquence nucléotidique codant pour un épitope du virus de la poliomyélite du type I, en particulier le peptide: Asp Asn Pro Ala Ser Thr Thr Asn Lys Asp Lys.

15. Souche de bactéries E. coli portant sur sa surface extérieure des récepteurs modifiés pour le phage λ contenant, exposés à la surface, plus particulièrement une séquence peptidique contenant un épitope caractéristique inséré entre deux acides aminés localisés entre les positions 140 et 160 ou 370 et 380 de la séquence d'acides aminés, et plus particulièrement aux positions 153 ou 374 de la protéine lamB.

16. Préparation immunogène dirigée contre des antigènes pathogènes neutralisables par des anticorps dirigés contre un épitope spécifique, caractérisée par la combinaison de bactéries selon la revendication 15, dans laquelle l'épitope remplit les conditions spécifiées cidessus, avec un véhicule pharmaceutique approprié pour la production de vaccins.

17. Protéine hybride comportant une enzyme contenant un épitope inséré dans une localisation de la chaîne peptidique de l'enzyme de telle sorte
   - que l'épitope soit exposé de telle sorte que, lorsque ladite protéine hybride est immunologiquement mise en contact avec des anticorps contre l'épitope, un complexe soit formé entre la protéine hybride et lesdits anticorps,
   - que l'activité enzymatique de ladite enzyme soit conservée dans la protéine hybride à l'état non-complexé,
   - que, lorsque ledit complexe est formé, ce complexe soit dépourvu de ladite activité enzymatique.

18. Protéine hybride comportant une enzyme contenant un épitope inséré dans celle-ci, caractérisée en ce que l'épitope est inséré dans une localisation de telle sorte
   - que l'épitope soit exposé de telle sorte que, lorsque ladite protéine hybride est immunologiquement mise en contact avec des anticorps contre l'épitope, un complexe soit formé entre ladite protéine hybride et lesdits anticorps,
   - que l'activité enzymatique de ladite enzyme soit inhibée dans la protéine hybride à l'état non-complexé,

20

- que, lorsque ledit complexe est formé, ce complexe présente une activité enzymatique.

19. Méthode pour réaliser le diagnostic du contenu éventuel d'anticorps dans un échantillon biologique comportant:
   - la mise en contact de la protéine hybride de la revendication 17 avec ledit échantillon biologique dans des conditions permettant la réaction immunologique entraînant la production d'un complexe immunologique entre les anticorps contre l'épitope et la protéine hybride portant l'épitope
   - la mise en contact du produit de réaction de ladite réaction immunologique avec le substrat hydrolysable par ladite enzyme de manière à permettre l'hydrolyse éventuelle dudit substrat
   - la détection de l'hydrolyse, s'il y en a, du substrat par des moyens physiques ou chimiques.

20. Méthode pour réaliser le diagnostic du contenu éventuel d'anticorps dans un échantillon biologique comportant:
   - la mise en contact de la protéine hybride de la revendication 18 avec ledit échantillon biologique dans des conditions permettant la réaction immunologique entraînant la production d'un complexe immunologique entre les anticorps contre l'épitope et la protéine hybride portant l'épitope
   - la mise en contact du produit de réaction de ladite réaction immunologique avec le substrat hydrolysable par ladite enzyme de manière à permettre l'hydrolyse éventuelle dudit substrat
   - la détection de l'hydrolyse, s'il y en a, du substrat par des moyens physiques ou chimiques.

FIG.1

# FIG.2 _ DNA SEQUENCES INSERTED IN PHASE AND AMINOACIDS SEQUENCES CORRESPONDING TO PEPTIDE VP1 93-1

93-103 VP1 = epitope of

asp asn pro ala ser thr thr asn lys asp lys

93  94  95  96  97  98  99  100 101 102 103

SYNTETIC NUCLEOTIDE INSERT SEQUENCE

asp asn  pro ala ser thr thr asn lys asp lys

GAT CCG | GAT AAC CCG GCG TCG ACC ACT AAC AAG GAT AAG
GC | CTA TTG  GGC CGC AGC TGG AGA TTG TTC CTA TTC | CTA G

Lam B

146

AJC 352    TCC | CGG
           AGG | GCC CTA  G

153

AJC 264  ...TCC | CGG
         ...AGG | GCC CTA  G

189

AJC 73   ...CTG | GGG CGG
         ...GAC | CCC GCC CTA  G

374

AJC 178  ...GAG | CGG
         ...CTC | GCC CTA  G

Lam B

147

GAT CCC GCC | TCT...
       GG CGG | AGA...

154

GAT CCC GCC | TCT...
       GG CGG | AGA...

198

GAT CCC GAC | TTG...
       GG CTG | AAC...

375

GAT CCC GAG | AAA...
       GG CTC | TTT...

23

# FIG. 3 — DNA AND AMINOACIDS SEQUENCES OF THEIR PHASE INTRODUCTION IN THE HYBRID PROTEIN OBTAINED FROM LAM B.

AJC 352

```
CGC TCC CGG GAT CCC GCC TCT GAA .....
arg ser arg asp pro ala  ser glu
145 146  Bam H1  site    147 146
```

AJC 264

```
...TCT TCC CGG GAT CCC GCC TCT TTC ......
ser ser arg asp pro ala  ser phe
152 153   Bam H1 site     154 155
```

AJC 73

```
.....GAA CTG GGG CGG GAT CCC GAC TTG CGT......
glu leu gly arg asp pro asp leu arg
188 189         Bam H1 site    198 199
```

AJC 353

```
.....CTG GGT GCG GGA TCC CGC GGT CGT ......
leu gly ala gly ser arg gly arg
189 190   Bam H1  site    194 195
```

AJC 178

```
___GAT GAG CGG GAT CCC GAG AAA TGG.....
asp glu arg asp pro glu lys trp
373 374   Bam H1  site  375 376
```

24

FIG.4